# EUROPÄISCHE PATENTSCHRIFT

(11) **EP 0 794 183 B1**
(45) Veröffentlichungstag und Bekanntmachung des Hinweises auf die Patenterteilung: **14.06.2000**
(21) Anmeldenummer: 97102726.3
(22) Anmeldetag: 20.02.1997
(51) Int. Cl.: C07D 307/79

(54) **(2,3-Dihydro-5-benzofuranyl)-acetonitril**
(2,3-Dihydro-5-benzofuranyl)-acetonitrile
(2,3-Dihydrobenzofuranyl)-acétonitrile

(30) Priorität: 05.03.1996 DE 19608408; 09.04.1996 DE 19614090
(43) Veröffentlichungstag der Anmeldung: 10.09.1997
(73) Patentinhaber: BAYER AG, 51368 Leverkusen (DE)
(72) Erfinder: Müller, Nikolaus, Dr., 40789 Monheim (DE); Steffan, Guido, Dr., 51519 Odenthal (DE)

(56) Entgegenhaltungen:
- EP-A- 0 029 360
- JOURNAL OF MEDICINAL CHEMISTRY, Bd. 29, Nr. 11, 1986, WASHINGTON DC, US, Seiten 2326-9, XP002033034 J. P. DUNN ET. AL.: "Analgetic and Antiinflammatory 7-Aroylbenzofuran-5-ylacetic Acids and 7-Aroylbenzothiophene-5-ylacetic Acids."
- JOURNAL OF MEDICINAL CHEMISTRY, Bd. 19, Nr. 2, Februar 1976, WASHINGTON DC, US, Seiten 303-8, XP000654744 N. HIROSE ET. AL.: "Antiinflammatory Activity of Some 2,3-Dihydrobenzofuran-5-acetic Acids and Related Compounds."

## Beschreibung

Die vorliegende Erfindung betrifft die neue Verbindung (2,3-Dihydro-5-benzofuranyl)acetonitril, ein Verfahren zu ihrer Herstellung und ihre Verwendung zur Herstellung von (2,3-Dihydro-5-benzofuranyl)-essigsäure, einem wichtigen Zwischenprodukt für die Herstellung verschiedener pharmazeutischer Wirkstoffe.

Es wurde die neue Verbindung (2,3-Dihydro-5-benzofuranyl)-acetonitril der Formel (I) gefunden 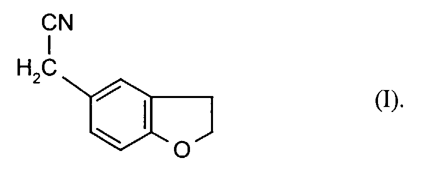 Es wurde weiterhin ein Verfahren zur Herstellung von (2,3-Dihydro-5-benzofuranyl)acetonitril gefunden, das dadurch gekennzeichnet ist, daß man 2,3-Dihydro-benzofuran in Gegenwart von überschüssigem 2,3-Dihydrobenzofuran, Alkoholen und/oder aromatischen Kohlenwasserstoffen als Lösungsmittel chlormethyliert, aus dem Reaktionsgemisch das Lösungsmittel und/oder überschüssiges 2,3-Dihydrobenzofuran abdestilliert, und das dadurch erhältliche rohe 2,3-Dihydro-5-chlormethylbenzofuran mit einem Cyanid zu (2,3-Dihydro-5-benzofuranyl)-acetonitril in Gegenwart von überschüssigem 2,3-Dihydro-5-chlormethyl-benzofuran, Alkoholen, Ketonen, Dimethyl-Sulfoxid oder Tetramethylensulfon als Lösungsmittel umsetzt. Das folgende Reaktionsschema illustriert das erfindungsgemäße Verfahren: 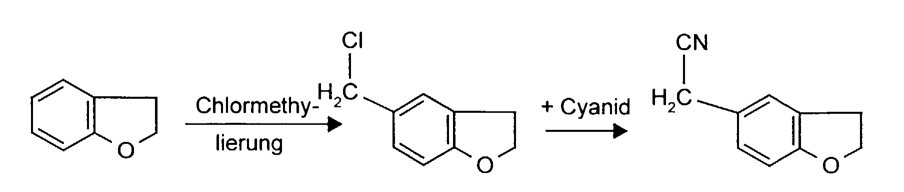 2,3-Dihydro-benzofuran ist beispielsweise aus Phenoxyethanol durch cyclisierende Dehydratisierung erhältlich (siehe z.B. J. Am. Chem. Soc. 41, 665 (1919)).

Unter "Chlormethylierung" wird die Einführung einer Cl-CH₂-Gruppe in ein Substrat verstanden. Im einfachsten Fall setzt man dabei das Substrat in Gegenwart eines Kondensationsmittels gleichzeitig mit Formaldehyd und Chlorwasserstoff um. Der Formaldehyd kann dabei z.B. als wäßrige Lösung zum Einsatz gelangen. Man kann auch Formaldehydderivate einsetzen, die unter Reaktionsbedingungen Formaldehyd freisetzen wie Formaldehydkondensationsprodukte (z.B. 1,3,5-Trioxan = Paraformaldehyd) und Acetale des Formaldehyds (z.B. Dimethylformal, Diethylformal, 1,3-Dioxan und 1,3-Dioxolan). Man kann auch mit speziellen Chlormethylierungsreagenzien arbeiten wie α,α'-Dichlordimethylether oder Chlormethylalkylethern der Formel Cl-CH₂-O-R mit R beispielsweise C₁-C₁₀-Alkyl. Beim Einsatz solcher spezieller Chlormethylierungsreagenzien kann gegebenenfalls auf den Zusatz von Chlorwasserstoff verzichtet werden. Ein Kondensationsmittel ist aber auch in diesem Fall einzusetzen.

Als Kondensationsmittel kommen z.B. Lewissäuren und organische und anorganische Protonensäuren infrage, beispielsweise Zinkchlorid, Eisenchlorid, Aluminiumchlorid, Zinntetrachlorid, Schwefelsäure, Phosphorsäure und Essigsäure.

Bezogen auf 1 Mol 2,3-Dihydrobenzofuran kann man in das erfindungsgemäße Verfahren z.B. 1 bis 10 Mol Formaldehyd oder soviel Formaldehyd lieferndes Formaldehydderivat, 1 bis 10 Mol Chlorwasserstoff (z.B. gasförmig oder als wäßrige Lösung) und 0,02 bis 1 Mol Kondensationsmittel einsetzen.

Wenn man mit speziellen Chlormethylierungsreagenzien arbeitet (siehe oben) können diese z.B: in Mengen von 1 bis 10 Molen pro Mol 2,3-Dihydrobenzofuran eingesetzt werden, wobei es nicht notwendig ist Chlorwasserstoff hinzuzufügen.

Die Chlormethylierung wird in überschüssigem 2,3-Dihydrobenzofuran durchgeführt werden. Es können aber auch Alkohole, aromatische Kohlenwasserstoffe oder Gemische solcher Lösungsmittel eingesetzt werden.

Die Reaktionstemperaturen können, je nach eingesetztem Chlormethylierungsreagenz und Kondensationsmittel, z.B. zwischen -10 und +150°C, bevorzugt zwischen 0 und 100°C, besonders bevorzugt zwischen 10 und 50°C, liegen.

Für die anschließende Umsetzung mit einem Cyanid wird ein rohes 2,3-Dihydro-5-chlormethylbenzofuran verwendet, das man erhält, wenn man das Lösungsmittel und/oder überschüssiges 2,3-Dihydrobenzofuran abdestilliert.

Als Cyanide kommen z.B. Alkali- und Erdalkalicyanide, Cyanide von Metallen der ersten und zweiten Nebengruppe des Periodensystems der Elemente und Ammonium- und Phosphoniumcyanide in Frage. Bevorzugt sind Natrium-, Kalium-, Zink-, Kupfer(I)- und Silbercyanid, insbesondere Natrium- und Kaliumcyanid.

Das Cyanid kann beispielsweise, bezogen auf 1 Mol, 2,3-Dihydro-5-chlormethylbenzofuran in einer Menge eingesetzt werden, die 1 bis 5 Äquivalenten Cyanid-Ionen entspricht. Vorzugsweise liegt die Menge bei 1 bis 1,5 Äquivalenten.

Die Reaktion mit dem Cyanid wird in überschüssigem 2,3-Dihydro-5-chlormethylbenzofuran, Alkoholen wie Methanol, Ethanol, Propanole und Butanole, Ketonen wie Aceton, Butanon-2, Methylisobutylketon und Cyclohexanon, Dimethylsulfoxid oder Tetramethylensulfon durchgeführt.

Die Reaktionsgeschwindigkeit kann durch den Zusatz von Metalliodiden, insbesondere Natrium- oder Kaliumiodid beträchtlich beschleunigt werden. Bezogen auf ein Äquivalent Cyanid kann man beispielsweise 0,001 bis 0,1 Mole eines einwertigen Iodids zusetzen.

Die Reaktionstemperaturen für die Umsetzung mit dem Cyanid können z.B. im Bereich von 0 bis 200°C liegen. Bevorzugt sind Temperaturen im Bereich von 20 bis 150°C, insbesondere im Bereich von 40 bis 100°C. Gegebenenfalls kann man die Reaktion in einem Autoklaven unter Druck durchführen, insbesondere dann, wenn man oberhalb des Siedepunktes eines eingesetzten Lösungsmittels arbeiten will.

Es ist vorteilhaft, die Umsetzung mit dem Cyanid in möglichst wasserfreiem Milieu durchzuführen.

Die Aufarbeitung des nach der Umsetzung mit dem Cyanid vorliegenden Reaktionsgemisches kann z.B. so erfolgen, daß man zunächst das ausgefallene Chlorid abtrennt, erst mit einem Lösungsmittel, vorzugsweise dem auch in der Umsetzung verwendeten wäscht, die Filtrate vereinigt, die Lösungsmittel daraus abzieht und den dabei erhaltenen Rückstand fraktioniert destilliert. Man erhält so (2,3-Dihydro-5-benzofuranyl)-acetonitril im allgemeinen in einer Ausbeute von über 60 % (bezogen auf 2,3-Dihydrobenzofuran).

(2,3-Dihydro-5-benzofuranyl)-acetonitril kann man gemäß an sich bekannten Verfahren zur Verseifung von Nitrilen in die entsprechende Säure überführen, im vorliegenden Fall ist das die (2,3-Dihydro-5-benzofuranyl)-essigsäure. Die Verseifung kann mit sauren oder alkalisch reagierenden Substanzen, vorzugsweise bei erhöhter Temperatur vorgenommen werden, im einfachsten Fall durch Erhitzen mit wäßriger Natronlauge unter Kochen am Rückfluß. Die Verseifung ist beendet wenn kein Ammoniak bzw. keine Ammoniumionen mehr gebildet werden. Aus dem Verseifungsgemisch kann man (2,3-Dihydro-5-benzofuranyl)-essigsäure isolieren, indem man es z.B. mit Wasser verdünnt, anschließend mit einer Säure, z.B. einer Mineralsäure versetzt und die dann ausfallende (2,3-Dihydro-5-benzofuranyl)-essigsäure durch Filtration isoliert.

Man kann so (2,3-Dihydro-5-benzofuranyl)-essigsäure im allgemeinen in Ausbeuten von über 85 % (bezogen auf das Nitril) erhalten.

(2,3-Dihydro-5-benzofuranyl)-essigsäure ist ein wichtiges Zwischenprodukt für die Herstellung von Pharmazeutika mit erzündungshemmender (siehe J. Med. Chem. 29, 2326-2329 (1986)), analgetischer und antipyretischer (siehe EP-OS 132 130) antimuscarinerger (siehe EP-OS 388 054) und antibakterieller Wirkung (siehe US-PS 4138 971). Der hier beschriebene Weg zur Herstellung von (2,3-Dihydro-5-benzofuranyl)-essigsäure über die neue Verbindung (2,3-Dihydro-5-benzofuranyl)-acetonitril stellt den mit Abstand kürzesten und effizientesten Weg für die Herstellung von (2,3-Dihydro-5-benzofuranyl)-essigsäure dar.

Die bekannten Verfahren zur Herstellung von (2,3-Dihydro-5-benzofuranyl)-essigsäure sind vielstufig (siehe US-PS 4 138 971) und/oder liefern das gewünschte Produkt nur in unzureichenden Ausbeuten. So verläuft der Weg über die Willgerodt-Reaktion (Keton + Ammoniumpolysulfid → Säureamid → Säure) nur in Gesamtausbeuten von weniger als 40 % und führt über geruchsintensive Schwefelverbindungen (siehe J. Med. Chem. 29, 2326-2329 (1986) und EP-OS 132 130). Das bekannte Verfahren zur Herstellung substituierter (2,3-Dihydro-5-benzofuranyl)-acetonitrile liefert diese in wesentlich geringeren Ausbeuten als das Verfahren der vorliegenden Erfindung unsubstituiertes (2,3-Dihydro-5-benzofuranyl)-acetonitril (siehe J. Med. Chem. 19, 303-308 (1976)).

### Beispiele

### Beispiel 1

120,2 g 2,3-Dihydrobenzofuran wurden in 500 ml Benzol gelöst und auf 0°C abgekühlt. Diese Lösung wurde unter Außenkühlung mit Chlorwasserstoffgas gesättigt. Unter kräftigem Rühren wurden nun 40 g Paraformaldehyd so eingetragen, daß die Innentemperatur unter 25°C gehalten werden konnte. Gleichzeitig wurde weiter Chlorwasserstoffgas eingeleitet. Nach vollendeter Paraformaldehydzugabe wurde noch eine Stunde bei 10°C Chlorwasserstoffgas eingeleitet und dann noch eine weitere Stunde bei Raumtemperatur nachgerührt. Dann wurde vom gebildeten Bodensatz dekantiert, die benzolische Lösung mit festem Natriumhydrogencarbonat entsäuert und das Benzol abdestilliert.

Der Rückstand wurde in 500 ml trockenem Aceton gelöst und mit 74 g trockenem Natriumcyanid und 5 g Natriumiodid versetzt. Unter Rühren und Feuchtigkeitsausschluß wurde 16 Stunden am Rückfluß erhitzt, abgekühlt und vom ausgefallenen Natriumchlorid abfiltriert. Das abfiltrierte Natriumchlorid wurde mit 250 ml Aceton nachgewaschen und aus den vereinigten Filtraten das Lösungsmittel abgezogen. Der Rückstand wurde durch fraktionierte Destillation im Vakuum aufgearbeitet. Die Fraktion mit einem Siedepunkt von 145-152°C bei 1,3 mbar wurde gesammelt und analysiert. Es wurden so 101 g (97,5 %iges) (2,3-Dihydro-5-benzofuranyl)-acetonitril erhalten. Das entspricht einer Ausbeute von 61,9 % der Theorie.
¹H-NMR-Spektrum: 3,2 ppm, (t, 2H), 3,65 ppm (s, 2H), 4,6 ppm (2H), 6,75 ppm (d, 1H), 7,0 ppm (2d, 1H) und 7,1 ppm (breites s, 1H).

### Beispiel 2

95,6 g des gemäß Beispiel 1 erhaltenen (2,3-Dihydro-5-benzofuranyl)-acetonitrils und 192 g 25 gew.-%ige Natronlauge wurden 3 Stunden lang am Rückfluß gekocht. Nach ca. 2 Stunden wurde das anfänglich zweiphasige Gemisch homogen. Nach Beendigung der Ammoniakentwicklung (nach rund 3 Stunden) wurde abgekühlt, mit 300 ml Wasser verdünnt und mit 61,5 g Schwefelsäure (96 Gew.-%ig) die entstandene Carbonsäure ausgefällt. Nach Abkühlen, Filtration, Waschen (3 mal mit je 100 ml Wasser) und Trocknen erhielt man 101 g 2,3-Dihydrobenzofuranyl-5-essigsäure mit einem Gehalt von 98,5 % und einem Schmelzpunkt von 96-98°C. Das entspricht einer Ausbeute von 93 % der Theorie.

Die so hergestellte (2,3-Dihydro-5-benzofuranyl)-essigsäure wies ein ¹H-NMR-Spektrum wie folgt auf: 3,1 ppm (t, 2H); 3,6 ppm (s, 2H); 4,5 ppm (t, 2H); 6,7 ppm (d, 1H); 7,0 ppm (d, 1H); 7,1 ppm (s, 1H).

Dieses Material erwies sich als identisch mit (2,3-Dihydro-5-benzofuranyl)-essigsäure, die gemäß der EP-OS 132 130 (siehe dort Präparationen 11-13) erhalten worden war.

## Patentansprüche

1. (2,3-Dihydro-5-benzofuranyl)-acetonitril der Formel (I)

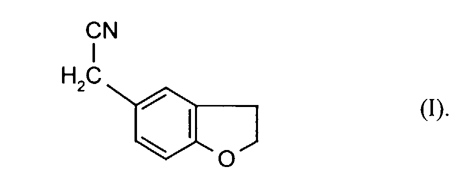

2. Verfahren zur Herstellung von (2,3-Dihydro-5-benzofuranyl)-acetonitril, dadurch gekennzeichnet, daß man 2,3-Dihydro-benzofuran in Gegenwart von überschüssigem 2,3-Dihydrobenzofuran, Alkoholen und/oder aromatischen Kohlenwasserstoffen als Lösungsmittel chlormethyliert, aus dem Reaktionsgemisch das Lösungsmittel und/oder überschüssiges 2,3-Dihydrobenzofuran abdestilliert, und das dadurch erhältliche rohe 2,3-Dihydro-5-chlormethylbenzofuran mit einem Cyanid zu (2,3-Dihydro-5-benzofuranyl)-acetonitril in Gegenwart von überschüssigem 2,3-Dihydro-5-chlormethyl-benzofuran, Alkoholen, Ketonen, Dimethyl-Sulfoxid oder Tetramethylensulfon als Lösungsmittel umsetzt..

3. Verfahren nach Anspruch 2, dadurch gekennzeichnet, daß man in die Chlormethylierung Formaldehyd oder ein Formaldehydderivat einsetzt, das unter Reaktionsbedingungen Formaldehyd freisetzt sowie Chlorwasserstoff und ein Kondensationsmittel.

4. Verfahren nach Ansprüchen 2 und 3, dadurch gekennzeichnet, daß man in die Chlormethylierung α,α'-Dichlordimethylether oder Chlormethylalkylether der Formel Cl-CH₂-O-R mit R = C₁-C₁₀-Alkyl einsetzt.

5. Verfahren nach Ansprüchen 2 bis 4, dadurch gekennzeichnet, daß man als Kondensationsmittel Lewissäuren oder organische oder anorganische Protonensäuren einsetzt.

6. Verfahren nach Ansprüchen 2 bis 5, dadurch gekennzeichnet, daß man auf 1 Mol 2,3-Dihydro-benzofuran 1 bis 10 Mol Formaldehyd oder soviel Formaldehyd lieferndes Formaldehydderivat, 1 bis 10 Mol Chlorwasserstoff und 0,02 bis 1 Mol Kondensationsmittel einsetzt.

7. Verfahren nach Ansprüchen 2 bis 6, dadurch gekennzeichnet, daß die Chlormethylierung zwischen -10 und +150°C durchführt.

8. Verfahren nach Ansprüchen 2 bis 7, dadurch gekennzeichnet, daß man als Cyanid ein Alkali- oder Erdalkalicyanid, ein Cyanid eines Metalls der ersten oder zweiten Nebengruppe des Periodensystem der Elemente oder ein Ammonium- oder ein Phosphoniumcyanid einsetzt.

9. Verfahren nach Ansprüchen 2 bis 8, dadurch gekennzeichnet, daß man der Umsetzung mit dem Cyanid ein Metalliodid zufügt.

10. Verfahren nach Ansprüchen 2 bis 9, dadurch gekennzeichnet, daß man die Umsetzung mit dem Cyanid bei 0 bis 200°C durchführt.

11. Verwendung von (2,3-Dihydro-5-benzofuranyl)-acetonitril zur Herstellung von (2,3-Dihydro-5-benzofuranyl)-essigsäure durch Verseifung.

## Claims

1. (2,3-dihydro-5-benzofuranyl)-acetonitrile of formula (I)

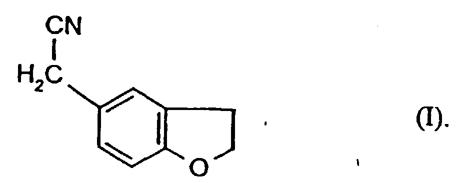

2. A method of producing (2,3-dihydro-5-benzofuranyl)-acetonitrile, characterised in that 2,3-dihydro-benzofuran is chloromethylated in the presence of excess 2,3-dihydrobenzofuran, alcohols and/or aromatic hydrocarbons as a solvent, the solvent and/or excess 2,3-dihydrobenzofuran is distilled off from the reaction mixture, and that the crude 2,3-dihydro-5-chloromethyl-benzofuran which is thereby obtainable is reacted with a cyanide to form (2,3-dihydro-5-benzofuranyl)-acetonitrile in the presence of excess 2,3-dihydro-5-chloromethyl-benzofuran, alcohols, ketones, dimethyl sulphoxide or tetramethylene sulphone as a solvent.

3. A method according to claim 2, characterised in that formaldehyde or a formaldehyde derivative which releases formaldehyde under the reaction conditions, as well as hydrogen chloride and a condensing agent, are used in the chloromethylation.

4. A method according to claims 2 and 3, characterised in that α,α'-dichlorodimethyl ether or a chloromethyl alkyl ether of formula Cl-CH₂-O-R where R = a C₁-C₁₀ alkyl, is used in the chloromethylation.

5. A method according to claims 2 to 4, characterised in that Lewis acids or organic or inorganic protonic acids are used as condensation agents.

6. A method according to claims 2 to 5, characterised in that 1 to 10 mol formaldehyde or an amount of a formaldehyde derivative which provides as much formaldehyde, 1 to 10 mol hydrogen chloride, and 0.02 to 1 mol condensation agent are used per 1 mol 2,3-dihydro-benzofuran.

7. A method according to claims 2 to 6, characterised in that chloromethylation is effected between -10 and +150°C.

8. A method according to claims 2 to 7, characterised in that an alkali or alkaline earth cyanide, a cyanide of a metal of the first or second subgroup of the periodic table of the elements, or an ammonium or phosphonium cyanide is used as a cyanide.

9. A method according to claims 2 to 8, characterised in that a metal iodide is added to the reaction with the cyanide.

10. A method according to claims 2 to 9, characterised in that the reaction with the cyanide is conducted at 0 to 200°C.

11. The use of (2,3-dihydro-5-benzofuranyl)-acetonitrile for the production of (2,3-dihydro-5-benzofuranyl)-acetic acid by saponification.

## Revendications

1. Le (2,3-dihydro-5-benzofurannyl)acétonitrile répondant à la formule (I)

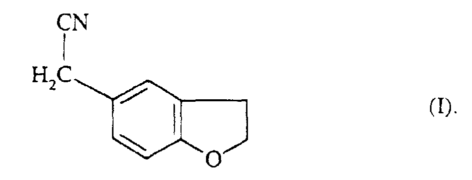

2. Procédé de préparation du (2,3-dihydro-5-benzofurannyl)-acétonitrile, caractérisé en ce que l'on soumet le 2,3-dihydrobenzofuranne à chlorométhylation en présence d'un excès de 2,3-dihydrobenzofuranne, d'un alcool et/ou d'un hydrocarbure aromatique qui sert de solvant, on distille le solvant et/ou l'excès de 2,3-dihydrobenzofuranne à partir du mélange de réaction, ce qui donne le 2,3-dihydro-5-chlorométhylbenzofuranne brut que l'on convertit par réaction avec un cyanure en le (2,3-dihydro-5-benzofurannyl)acétonitrile en présence d'un excès de 2,3-dihydro-5-chlorométhylbenzofuranne, d'un alcool, d'une cétone, du diméthylsulfoxyde ou de la tétraméthylénesulfone, qui sert de solvant.

3. Procédé selon la revendication 2, caractérisé en ce que l'on utilise à la chlorométhylation du formaldéhyde ou un dérivé du formaldéhyde libérant ce composé dans les conditions de la réaction, ainsi que du chlorure d'hydrogène et un agent de condensation.

4. Procédé selon les revendications 2 et 3, caractérisé en ce que l'on met en oeuvre à la chlorométhylation l'éther α,α'-dichlorodiméthylique ou un éther chlorométhylalkylique de formule Cl-CH₂-O-R dans laquelle R représente un groupe alkyle en C₁-C₁₀.

5. Procédé selon les revendications 2 à 4, caractérisé en ce que l'on utilise en tant qu'agent de condensation un acide de Lewis ou un acide protonique, organique ou minéral.

6. Procédé selon les revendications 2 à 5, caractérisé en ce que, pour 1 mol de 2,3-dihydrobenzofuranne, on met en oeuvre 1 à 10 mol de formaldéhyde ou d'un dérivé du formaldéhyde libérant ce composé, 1 à 10 mol de chlorure d'hydrogène et 0,02 à 1 mol d'agent de condensation.

7. Procédé selon les revendications 2 à 6, caractérisé en ce que la chlorométhylation est réalisée à des températures allant de -10 à +150°C.

8. Procédé selon les revendications 2 à 7, caractérisé en ce que le cyanure utilisé est un cyanure alcalin ou alcalino-terreux, un cyanure d'un métal du premier ou du deuxième sous-groupe de la classification périodique des éléments ou un cyanure d'ammonium ou de phosphonium.

9. Procédé selon les revendications 2 à 8, caractérisé en ce que, à la réaction avec le cyanure, on ajoute un iodure métallique.

10. Procédé selon les revendications 2 à 9, caractérisé en ce que la réaction avec le cyanure est réalisée à des températures de 0 à 200°C.

11. Utilisation du (2,3-dihydro-5-benzofurannyl)acétonitrile pour la préparation de l'acide (2,3-dihydro-5-benzofurannyl)acétique par saponification.
